# EUROPÄISCHE PATENTSCHRIFT

(11) **EP 1 403 390 B1**
(45) Veröffentlichungstag und Bekanntmachung des Hinweises auf die Patenterteilung: **23.08.2006**
(21) Anmeldenummer: 03020304.6
(22) Anmeldetag: 09.09.2003
(51) Int. Cl.: C22C 27/02, A61L 31/00

(54) **Nb-Ta-Zr Legierung zur Verwendung für Stents**
A Nb-Zr-Ta Alloy for use in the manufacture of Stents
Alliage de Nb-Ta-Zr pour la fabrication de Stents

(30) Priorität: 27.09.2002 DE 10245516
(43) Veröffentlichungstag der Anmeldung: 31.03.2004
(73) Patentinhaber: W.C. Heraeus GmbH, 63450 Hanau (DE)
(72) Erfinder: Trötzschel, Jens, 63486 Bruchköbel (DE); Wachter, Hans-Jürgen, Dr., 63322 Rödermark (DE); Krüger, Frank, Dr., 63486 Bruchköbel (DE); Frericks, Matthias, 63456 Hanau (DE)
(74) Vertreter: Kühn, Hans-Christian

(56) Entgegenhaltungen:
- WO-A-02/05863
- WO-A-02/43787
- GB-A- 933 712

## Beschreibung

Die Erfindung betrifft Legierungen für Stents, die im aus Niob (Nb), Tantal (Ta) und Zirkonium (Zr) bestehen, sowie Stents aus solchen Legierungen.

Stents sind an sich bekannt und werden z.B. in US-A-5,628,787, US-A-5,630,840 und EP-A-0 873 734 beschrieben. Es wurden neben Edelstahl, Nickel- und Tantallegierungen auch Niob-Zirkoniumlegierungen für Stents vorgeschlagen, wobei der Nb-Gehalt bei über 90 %, vorzugsweise über 95 bzw. 98 % liegt (WO 02/43787)

GB 933 712 A beschreibt hoch warmfeste Nb-Ta Legierungen mit 15 - 75 % Ta, 17 - 75 % Nb und mindestens einem weiteren Metall der Gruppe Zr, W, Mo und Ta, wobei Zr zu 0.245 - 6 % enthalten sein kann.

Es stellt sich die Aufgabe, Legierungen bereitzustellen, die den hohen Anforderungen in Bezug auf Biokompatibilität genügen, keine bekannten Allergene enthalten, gute mechanische Eigenschaften aufweisen, und die im Röntgenbild sichtbar und in der Kernspintomographie identifizierbar sind.

Es wurde nun gefunden, dass Stents aus den weiter unten beschriebenen Legierungen von Nb, Ta und Zr überraschenderweise die folgenden Eigenschaften in sich vereinigen: Gute Biokompatibilität bei sehr guten mechanischen Eigenschaften, Röntgenopazität und Identifizierbarkeit im Kernspintomographen auf Grund der magnetischen Eigenschaften. Problematische Bestandteile wie Nickel sind nicht vorhanden, die Röntgenopazität ist nicht zu groß wie bei Legierungen mit sehr hohem Ta-Anteil, und die Identifizierbarkeit mit Kernspinverfahren ist bei der wachsenden Verbreitung dieser Methode ein großer Vorteil.

Die beanschprüchte Legierung hat folgende Anteile:

| | | | |
|---|---|---|---|
| 4. | 80 - 90 % Nb, | 0,5 - 5 % Zr, | 5 -19,5 % Ta. |

Besonders vorteilhaft sind Legierungen mit einem Niobgehalt zwischen 80 und 90 %. Dabei wirken sich die Erhöhung der Röntgensichtbarkeit und die Verbesserung der mechanischen Eigenschaften aus - mit der Möglichkeit, den Querschnitt der Verbindungsstege des Stents zu reduzieren.

Legierungen, bei denen mehr Tantal als Zirkonium vorhanden ist, sind ebenfalls bevorzugte Ausführungsformen der Erfindung.

Beispiele für besonders bevorzugte Legierungen sind:

| | | | |
|---|---|---|---|
| 3. | 90 % Nb, | 1 % Zr, | 9 % Ta. |
| 4. | 85 % Nb, | 1 % Zr, | 14 % Ta. |

Die Erfindung betrifft auch Stents aus den beschriebenen Legierungen, insbesondere Stents, bei denen die Verbindungsstege einen relativ geringen Querschnitt besitzen.

Die Fertigung der Legierungen und der Stents daraus erfolgen nach geläufigen, dem Fachmann bekannten Verfahren.

## Patentansprüche

1. Legierung, bestehend aus
80 - 90 % Nb.
0.5 - 5 % Zr,
5 - 19.5 % Ta.

2. Stent aus einer Legierung bestehend aus
80 - 90 % Nb.
0.5 - 5 % Zr,
5 - 19.5 % Ta.

## Claims

1. Alloy, consisting of
80 - 90 % Nb.
0.5 - 5 % Zr,
5 - 19.5 % Ta.

2. Stent of alloy consisting of
80 - 90 % Nb.
0.5 - 5 % Zr,
5 - 19.5 % Ta.

## Revendications

1. L'alliage se compose de
80-90 % Nb.
0.5 - 5 % Zr,
5 - 19.5 % Ta.

2. Stent à base d'un alliage se compose de
80 - 90 % Nb.
0.5 - 5 % Zr,
5 - 19.5 % Ta.
